# EUROPÄISCHE PATENTSCHRIFT

(11) **EP 3 521 441 B1**
(45) Veröffentlichungstag und Bekanntmachung des Hinweises auf die Patenterteilung: **18.03.2020**
(21) Anmeldenummer: 18212478.4
(22) Anmeldetag: 13.12.2018
(51) Int. Cl.: C12Q 1/04, C12Q 3/00, G01N 33/487, G01N 33/00, G01N 7/00, G01N 33/32

(54) **MESSGERÄT UND VERFAHREN ZUR BESTIMMUNG DES VERKEIMUNGSGRADES VON PROZESSFLÜSSIGKEITEN**
MEASURING DEVICE AND METHOD FOR DETERMINING THE CONTAMINATION LEVEL OF PROCESS FLUIDS
APPAREIL DE MESURE ET PROCÉDÉ DE DÉTERMINATION DU DEGRÉ DE PROLIFÉRATION DE GERMES DES LIQUIDES DE TRAITEMENT

(30) Priorität: 06.02.2018 DE 102018102658
(43) Veröffentlichungstag der Anmeldung: 07.08.2019
(73) Patentinhaber: AdvaTec Projects GmbH & Co. KG, 52525 Heinsberg (DE)
(72) Erfinder: MAUS, Michael, D-72160 Horb a.N. (DE); WIMMER, Thomas, D-52525 Heinsberg (DE); BERNER, Ulrich, D-73257 Köngen (DE); JOSUPEIT, Frank, D-71088 Holzgerlingen (DE)
(74) Vertreter: Geitz Truckenmüller Lucht Christ

(56) Entgegenhaltungen:
- EP-A1- 2 833 117
- US-A- 3 740 320
- US-A- 4 220 715
- US-A- 4 330 385
- US-A- 4 546 640
- US-A- 4 650 767
- US-A- 5 421 194
- US-A1- 2015 099 274
- US-B1- 6 420 187

## Beschreibung

Die Erfindung betrifft ein Messgerät und ein Verfahren zur Bestimmung des Verkeimungsgrades von Prozessflüssigkeiten.

In den Prozessbädern heutiger Industrieanlagen treten immer wieder hohe Keimzahlen auf. Zur Bestimmung der Keimzahlen werden Nährböden mit Prozessflüssigkeit inkubiert und im Anschluss im Inkubator bebrütet. Nach etwa zwei Tagen wird die Zellzahl durch Auszählung der gewachsenen Zellkolonien bestimmt. Diese Vorgehensweise ist notwendig, um die Prozessstabilität zu gewährleisten. Bei stark erhöhten Keimzahl-Werten von >10⁴ KBE/ml (KBE = "kolonienbildende Einheiten" kann es zu massiven Prozessstörungen kommen, die im Allgemeinen durch Zugabe von Bioziden unterbunden werden müssen. Alternativ werden Proben aus Bädern entnommen und an ein akkreditiertes Labor oder Institut versandt, das den Befund nach frühestens drei Tagen an die Betreiber der Anlage übermittelt.

In der exponentiellen Wachstumsphase (Verdopplungsraten -100 min) der Keime sind die oben genannten Methoden zu langsam. Biozid kann nicht immer rechtzeitig zu dosiert werden, so dass es zu Qualitätseinbußen in der Produktion kommt. Kann die Chemie der Prozessbäder nicht mehr regeneriert werden, müssen diese zumindest teilweise verworfen werden. Dies ist sehr teuer und belastet die Umwelt zusätzlich.

Die meisten Verfahren zur Keimzahlbestimmung funktionieren über einen Bypass, wo eine Flüssigkeit entweder in einen Behälter oder direkt zu einer Art von Sensorik zugeführt wird, wie z.B. in der DE 199 21 999 A1 beschrieben. Dies birgt sehr große Nachteile und Risiken im robusten Gebrauch in Industrieprozessbädern. Zum einen können im Falle von aggressiven Medien die Aufbauten (Leitungen, Pumpen Ventile etc.) leicht beschädigt werden, so dass ihre Standzeit sehr kurz ist. Zum anderen können durch Ablagerung und Verkeimungen des Messaufbaus die Ergebnisse stark verfälscht werden. Spülvorrichtungen können hier Abhilfe schaffen, sind aber sowohl in der Anschaffung als auch in der Instandhaltung sehr kostenintensiv. Im Falle von KTL-Lacken (KTL = kathodische Tauchlackierung) werden solche Aufbauten nahezu unmöglich, da diese Prozessflüssigkeit zur Koagulation und Sedimentation neigt.

In absolut klaren Prozessbädern kann die Keimzahl auch durch Zellzähler ("Zellcounter") online bestimmt werden. Diese Methode hat den Nachteil, dass auch tote Zellen und Schmutzpartikel mitgezählt werden.

Eine andere schnelle Methode zur Bestimmung des Verkeimungsgrades ist die Bestimmung des ATP-Gehalts (ATP = Adenintriphospat). Auch hier werden tote Zellen miterfasst. Des Weiteren ist eine Probennahme mit anschließender spektroskopischer Auswertung des ATP-Gehalts im Labor notwendig, so dass man nicht von einer "online-Methode" sprechen kann.

Aus der US 4 330 385 A ist ein Gerät zur Messung der in einer Flüssigkeit gelösten Sauerstoffmenge bekannt geworden, das besonders nützlich zur Messung der Menge an gelösten Sauerstoff in stark kontaminierten Flüssigkeiten wie Abwasser ist.

Es ist eine Aufgabe der Erfindung, ein Messgerät und ein Verfahren der eingangs genannten Art zur Verfügung zu stellen, mit denen eine Online-Erfassung des Verkeimungsgrades ("Online-Keimmonitoring") von beliebigen Prozessflüssigkeiten ermöglicht und nach vergleichsweise kurzer Zeit (wenigen Stunden) ein zuverlässiger Signal-Wert in Korrelation zur Keimzahl verfügbar ist. Zudem soll das Messgerät und soll die Anwendung des Verfahrens robust für nahezu alle flüssigen Medien sein, wie sie in belüfteten Industriebädern vorkommen.

Diese Aufgabe wird hinsichtlich des Messgeräts insbesondere durch die Merkmale des Anspruches 1 und hinsichtlich des Verfahrens unter Verwendung dieses Messgeräts wie in Anspruch 8 angegeben gelöst.

Demgemäß betrifft die Erfindung ein Messgerät zur Bestimmung des Verkeimungsgrades von Prozessflüssigkeiten, das wenigstens einen Gas-Sensor zur Messung der Gaskonzentration eines von etwaig oder tatsächlich in der Prozessflüssigkeit enthaltenen aeroben Keimen produzierbaren oder produzierten Gases und eine damit verbundene Auswertevorrichtung zur Auswertung eines von dem Gas-Sensor erzeugten, vorzugsweise elektrischen, Sensorsignals umfasst, das mit dem Verkeimungsgrad korreliert, wobei ein einen Eintauch-Hohlraum aufweisender, vorzugsweise rotationssymmetrisch gestalteter, geometrischer Gasauffang-Hohlkörper vorgesehen ist, der in die Prozessflüssigkeit derart teilweise eintauchbar ist oder eingetaucht ist, dass in dem Gasauffang-Hohlkörper unmittelbar oberhalb einer sich in dem Eintauch-Hohlraum befindlichen Flüssigkeitsoberfläche der Prozessflüssigkeit ein auch mit Überstand bezeichneter Gasauffang-Hohlraum zum Auffangen des aus der Prozessflüssigkeit in die Umgebung austretenden bzw. ausgetretenen Gases ausgebildet ist, und dass eine Gas-Zuführleitung vorgesehen ist, deren eines Zuführleitungsende in den Gasauffang-Hohlraum des Gasauffang-Hohlkörpers mündet und deren anderes Zuführleitungsende in einen Vorrichtungs-Hohlraum einer den wenigstens einen Gas-Sensor, insbesondere eines Sensorarrays, zum Messen der Konzentration des aus der Prozessflüssigkeit ausgetretenen Gases enthaltenden Sensor-Vorrichtung mündet, der in Gasverbindung mit dem wenigstens einem Gas-Sensor steht, und dass eine ausschließlich an ihren beiden Rückführleitungsenden nach außen offene Gas-Rückführleitung vorgesehen ist, deren eines Rückführleitungsende in den Vorrichtungs-Hohlraum der Sensor-Vorrichtung mündet und deren anderes Rückführleitungsende in den Gasauffang-Hohlraum des Gasauffang-Hohlkörpers mündet, und dass eine Gas-Pumpe zum Pumpen des Gases von dem Gasauffang-Hohlraum des Gasauffang-Hohlkörpers über den wenigstens einen Gas-Sensor zurück in den Gasauffang-Hohlraum des Gasauffang-Hohlkörpers vorgesehen ist.

Gemäß einer besonders bevorzugten Ausgestaltung des Messgeräts kann vorgesehen sein, dass der Gasauffang-Hohlkörper zylinderförmig, glockenförmig oder trichterförmig oder als Zylinder oder als Glocke oder als Trichter gestaltet ist. Insbesondere dann, wenn der geometrische Hohlkörper als eine, vorzugsweise trichterförmige, Glocke oder als ein Trichter gestaltet ist, kann eine deutliche Erhöhung des Sensorsignals und der Detektionsgrenze erreicht werden.

Gemäß einer ganz besonders bevorzugten Ausführungsvariante des Messgeräts kann vorgesehen sein, dass ein erster Rührer zur Homogenisierung der Prozessflüssigkeit und/oder ein zweiter Rührer zum Austreiben des von den aeroben Keimen produzierten und in der Prozessflüssigkeit gelösten Gases und/oder zum Austreiben des von den aeroben Keimen produzierten und an in der Prozessflüssigkeit enthaltenen Festkörperpartikeln adsorbierten Gases vorgesehen ist. Bevorzugt können der erste Rührer und der zweite Rührer in einen gemeinsamen Rührer integriert sein bzw. kann ein einziger Rührer für die vorgennannten Funktionen vorgesehen sein. Durch diese Maßnahmen sind besonders kurze Messzeiten bzw. schnelle Messungen des Verkeimungsgrades möglich.

Gemäß einer Weiterbildung des Messgeräts kann vorgesehen sein, dass eine steuerbare Hebe- und Senkvorrichtung zum Anheben und Absenken des Gasauffang-Hohlkörpers, ggf. auch des Rührers, vorgesehen ist, mittels welcher der Gasauffang-Hohlkörper, ggf. auch der Rührer, von einer Anhebestellung außerhalb der Prozessflüssigkeit in eine Absenkstellung bewegbar ist, in welcher der Gasauffang-Hohlkörper teilweise, ggf. auch der Rührer zumindest teilweise oder ganz, in die Prozessflüssigkeit eingetaucht ist und in welcher die Gaskonzentration eines von den Keimen produzierten Gases mittels des wenigstens einen Gas-Sensors messbar ist oder gemessen wird und mittels welcher der Gasauffang-Hohlkörper, ggf. auch der Rührer, von der Absenkstellung in die Anhebestellung bewegbar ist. Dabei kann vorgesehen sein, dass die Hebe- und Senkvorrichtung einen Abstands-Sensor zum Messen des Abstandes des Gasauffang-Hohlkörpers, insbesondere dessen unteren Randes, zu der Flüssigkeitsoberfläche der Prozessflüssigkeit aufweist. Bevorzugt kann es sich bei dem Abstands-Sensor um einen Ultraschall-Sensor handeln. Durch diese Maßnahmen können noch bessere, automatisierte Messungen durchgeführt werden.

Gemäß einer vorteilhaften Weiterbildung des Messgeräts kann vorgesehen sein, dass die Auswertevorrichtung mit einer Schnittstelle zum Anschließen einer Steuerung versehen ist, mittels welcher ein Biozid in Abhängigkeit von der mittels des wenigstens einen Gas-Sensors gemessenen Gaskonzentration des von den Keimen über der Zeit produzierten Gases in die Prozessflüssigkeit dosierbar ist oder dass die Auswertevorrichtung direkt oder über die Schnittstelle mit einer Steuerung verbunden ist, mittels welcher ein Biozid in Abhängigkeit von der mittels des wenigstens einen Gas-Sensors gemessenen Gaskonzentration des von den Keimen über der Zeit produzierten Gases in die Prozessflüssigkeit dosierbar ist. Die Steuerung ermöglicht die industrielle Umsetzung einer vollautomatischen Bioziddosierung in Abhängigkeit von der gemessenen Keimkonzentration in der Prozessflüssigkeit. Wenn das Messgerät die Schnittstelle zum Anschluss der Steuerung aufweist, kann das Messgerät optional ohne oder mit der über die Schnittstelle angeschlossenen Steuerung eingesetzt werden.

Das erfindungsgemäße Messgerät kann alle aeroben Keime erfassen und online ein Signal ausgeben, welches mit der Keimzahl korreliert.

Die Erfindung betrifft auch ein Verfahren zur Bestimmung des Verkeimungsgrades von Prozessflüssigkeiten mittels des erfindungsgemäßen Messgeräts, wobei die Gaskonzentration eines Gases, das von etwaig oder tatsächlich in einer Prozessflüssigkeit enthaltenen Keimen produzierbar ist oder produziert wird, mittels des wenigstens einen Gas-Sensors gemessen wird, der ein, vorzugsweise elektrisches, Sensorsignal erzeugt, das mit dem Verkeimungsgrad korreliert, wobei das Sensorsignal mittels der Auswertevorrichtung ausgewertet wird, wobei der Gasauffang-Hohlkörper in die Prozessflüssigkeit derart teilweise eingetaucht ist, dass in dem Gasauffang-Hohlkörper unmittelbar oberhalb der sich in dem Eintauch-Hohlraum befindlichen Flüssigkeitsoberfläche der Prozessflüssigkeit der auch mit Überstand bezeichnete Gasauffang-Hohlraum zum Auffangen des aus der Prozessflüssigkeit in die Umgebung austretenden Gases ausgebildet ist, wobei das aus der Prozessflüssigkeit in die Umgebung ausgetretene Gas in dem Gasauffang-Hohlraum aufgefangen und von dort mittels der Gas-Pumpe über die Gas-Zuführleitung dem wenigstens einen Gas-Sensor zugeführt, über den wenigstens einen Gas-Sensor geleitet und über die Gas-Rückführleitung wieder in den Gasauffang-Hohlraum des Gasauffang-Hohlkörpers zurück gepumpt wird.

Gemäß einer besonders bevorzugten Ausgestaltung des Verfahrens kann vorgesehen sein, dass der Gasauffang-Hohlkörper zylinderförmig, glockenförmig oder trichterförmig oder als Glocke oder als Trichter oder als Zylinder gestaltet ist. Insbesondere dann, wenn der geometrische Hohlkörper als eine, vorzugsweise trichterförmige, Glocke oder als ein Trichter gestaltet ist, kann eine deutliche Erhöhung des Sensorsignals und der Detektionsgrenze erreicht werden.

Gemäß einer ganz besonders bevorzugten Ausführungsvariante des Verfahrens kann vorgesehen sein, dass das von den aeroben Keimen produzierte und in der Prozessflüssigkeit gelöste Gas und/oder dass das von den aeroben Keimen produzierte und an in der Prozessflüssigkeit enthaltenen Festkörperpartikeln adsorbierte Gas, insbesondere diskontinuierlich, vorzugsweise in periodischen Abständen, mittels eines Rührers ausgetrieben wird, wobei die Gaskonzentration des ausgetriebenen Gases mittels des wenigstens einen Gas-Sensors gemessen wird. Durch diese Maßnahmen sind besonders kurze Messzeiten bzw. schnelle Messungen des Verkeimungsgrades möglich.

Gemäß einer Weiterbildung des Verfahrens kann vorgesehen sein, dass der Gasauffang-Hohlkörper, ggf. auch der Rührer, mittels einer steuerbaren Hebe- und Senkvorrichtung von einer Absenkstellung, in welcher der Gasauffang-Hohlkörper teilweise, ggf. auch der Rührer zumindest teilweise, in die Prozessflüssigkeit eingetaucht ist und in welcher die Gaskonzentration des von den Keimen produzierten Gases mittels des wenigstens einen Gas-Sensors gemessen wird, in eine Anhebestellung überführbar ist oder überführt wird, und umgekehrt, in welcher sich der Gasauffang-Hohlkörper, ggf. auch der Rührer, außerhalb der Prozessflüssigkeit befindet. Ferner kann vorgesehen sein, dass der Gasauffang-Hohlkörper, ggf. auch der Rührer, bevor er sich in einer oder seiner Absenkstellung befindet, mittels einer oder der steuerbaren Hebe- und Senkvorrichtung, ausgehend von einer oder der Anhebestellung, in welcher er sich außerhalb der Prozessflüssigkeit befindet, in eine Absenkstellung bzw. in die Absenkstellung bewegt wird, in welcher der Gasauffang-Hohlkörper teilweise, ggf. auch der Rührer zumindest teilweise, in die Prozessflüssigkeit eingetaucht ist. Durch diese Maßnahmen können noch bessere, automatisierte Messungen durchgeführt werden.

Gemäß einer vorteilhaften Weiterbildung des Verfahrens kann vorgesehen sein, dass die Auswertevorrichtung direkt oder über eine Schnittstelle mit einer Steuerung verbunden ist, mittels welcher ein Biozid in Abhängigkeit von der mittels des wenigstens einen Gas-Sensors gemessenen Gaskonzentration des von den Keimen über der Zeit produzierten Gases in die Prozessflüssigkeit dosiert wird. Die Steuerung ermöglicht die industrielle Umsetzung einer vollautomatischen Bioziddosierung in Abhängigkeit von der gemessenen Keimkonzentration in der Prozessflüssigkeit.

Im erfindungsgemäßen Verfahren ist vorgesehen, dass der Verkeimungsgrad mittels des Messgeräts nach einem der Ansprüche 1 bis 7 bestimmt wird.

Mit dem erfindungsgemäßen Messgerät und mit dem erfindungsgemäßen Verfahren ist eine kontinuierliche, also unterbrechungsfreie Prozesskontrolle bezüglich der Bestimmung des Verkeimungsgrades durch etwaig in der Prozessflüssigkeit enthaltene Keime möglich.

Im Zusammenhang mit der vorliegenden Erfindung wird unter Online-Erfassung des Verkeimungsgrades ("Online-Keimmonitoring") verstanden, dass der Messwert vollautomatisch erzeugt wird, also ohne dass eine Verprobung erforderlich ist.

Weitere Vorteile, Merkmale und Gesichtspunkte der Erfindung gehen aus den Ansprüchen und aus dem nachfolgenden Beschreibungsteil hervor, in dem ein bevorzugtes Ausführungsbeispiel der Erfindung anhand der Figuren beispielhaft beschreiben ist. Es zeigen:
- Figur 1: einen schematischen Aufbau einer Anlage mit einem erfindungsgemäßen Messgerät;
- Figur 2: vier im Abstand von jeweils etwa 24h durchgeführte Langzeitmessungen.

Das Messgerät 10 besteht im Wesentlichen aus einer auch als Gasauffang-Hohlkörper bezeichneten Glocke 15, einer Sensor-Vorrichtung 20 mit einem Gassensorarray 12 mit mindestens einem Gas-Sensor, einer Gas-Pumpe 14 und einer Auswerteeinheit 13. Die Auswerteeinheit 13 kann eine Schnittstelle (Interface) 25 zu einer Anlagensteuerung 26 besitzen. Die Glocke 15 weist bevorzugt die Form eines Trichters auf, sie kann jedoch auch die Form eines Zylinders oder eines sonstigen geometrischen Hohlkörpers haben. Die Glocke 15 weist in ihrer bestimmungsgemäßen Gebrauchsstellung betrachtet einen oberen, vorzugsweise zylinderförmigen oder zylindrischen Hals 27 auf. Der Hals 27 geht nach unten in einen sich nach unten erweiternden, nach unten offenen Trichter 28 über. Der Trichter 28 weist eine kegelförmige Trichterwandung 29 auf. Der Trichter 28 weist an seinem unteren Ende einen umlaufenden, vorzugsweise kreisförmigen, Rand 30 auf.

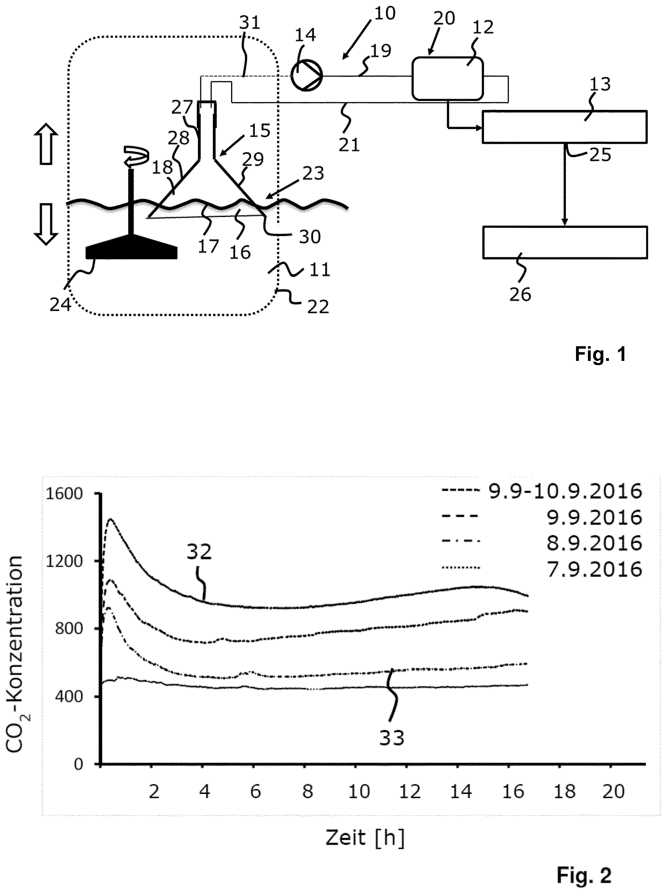

Für den Messbetrieb wird die trichterförmige Glocke 15, ggf. zusammen mit dem Rührer 24 mittels einer steuerbaren Hebe- und Senkvorrichtung 22, die einen Ultraschallsensor umfasst - oder manuell - in eine untere Absenkstellung 27 überführt, in der die Glocke 15 mit ihrem unteren Rand 30 teilweise in die Prozessflüssigkeit eintaucht. Ggf. wird gleichzeitig oder später auch das Rührelement des Rührers 24 mittels einer oder der steuerbaren Hebe- und Senkvorrichtung 22 in die Prozessflüssigkeit eingetaucht (Figur 1). In der so eingetauchten Absenkstellung 27 ist in der Glocke 15 unmittelbar oberhalb der sich in dem auch mit Eintauch-Hohlraum bezeichneten Inneren des Trichters 28 befindlichen Flüssigkeitsoberfläche 17 der Prozessflüssigkeit 11 ein auch mit Überstand bezeichneter Gasauffang-Hohlraum 18 zum Auffangen des aus der Prozessflüssigkeit 11 in die Umgebung ausgetretenen Gases ausgebildet.

Wenn die Prozessflüssigkeit 11 aerobe Keime enthält, produzieren diese Gase, beispielsweise Kohlendioxid (CO₂). Ein Teil dieses Kohlendioxids wandert nach oben zu der Flüssigkeitsoberfläche 17 und tritt aus dieser in den Gasauffang-Hohlraum 18 der Glocke 15 aus. Das darin eingeschlossene Gasvolumen wird durch eine Gas-Pumpe 2 im Kreislauf über das Gassensorarray 12 geleitet, welches ein elektrisches Signal generiert. Das Gassensorarray 12 kann mit allen gebräuchlichen Sensortypen bestückt sein. Das Gassensorarray 12 muss mit mindestens einem Gas-Sensor bestückt sein, mittels dessen die Konzentration des von den Keimen produzierten Gases messbar ist. Die von dem wenigstens einen Gas-Sensor bzw. von dem Gassensorarray 12 erzeugten Signale werden an die Auswerteeinheit 13 mit intelligenter Algorithmik und optionaler Visualisierung der Messergebnisse wie PC, Mikrocontroller, Datenlogger, etc. mit optionaler Schnittstelle 25 zu der optionalen Anlagensteuerung 26 weitergeleitet.

Das in dem Gasauffang-Hohlraum 18 der Glocke 15 eingeschlossene Gasvolumen wird mittels der Gas-Pumpe 14 durch eine Gas-Zuführleitung 19 gepumpt, deren eines Zuführleitungsende in den Gasauffang-Hohlraum 18 der Glocke 15 mündet und deren anderes Zuführleitungsende in einen Vorrichtungs-Hohlraum des den wenigstens einen Gas-Sensor 12 enthaltenden Sensor-Vorrichtung 20 mündet. Der besagte Vorrichtungs-Hohlraum steht in Gasverbindung mit dem wenigstens einem Gas-Sensor 12.

Außerdem ist eine ausschließlich an ihren beiden Rückführleitungsenden nach außen offene Gas-Rückführleitung 21 vorgesehen, deren eines Rückführleitungsende in den Vorrichtungs-Hohlraum der Sensor-Vorrichtung mündet und deren anderes Rückführleitungsende in den Gasauffang-Hohlraum 18 der Glocke 15 mündet.

Die Gas-Pumpe 14 dient zum Pumpen des Gases von dem Gasauffang-Hohlraum 18 der Glocke 15 über den wenigstens Gas-Sensor 12 zurück in den Gasauffang-Hohlraum 18 der Glocke 15 in einem Kreislaufbetrieb.

Falls es sich bei dem Messgerät um ein Handgerät handelt, kann die Schnittstelle zu einem PC oder zu einem sonstigen Aufzeichnungsgerät, wie SD-Karte, USB etc. ausgelegt sein.

Optional ist eine Apparatur mit einem Rührer 24 zur Homogenisierung der Prozessflüssigkeit ausgestattet. Dieser Rührer 24 kann auch besonders vorteilhaft zum Austreiben des von den aeroben Keimen produzierten und in der Prozessflüssigkeit 11 gelösten Gases und/oder zum Austreiben des von den aeroben Keimen produzierten und an in der Prozessflüssigkeit 11 enthaltenen Festkörperpartikeln adsorbierten Gases eingesetzt werden.

Zur Entfeuchtung des von den Keimen produzierten Gases, also des Messgases, kommt ein Schlauch 31 zum Einsatz, der für Wasserdampf durchlässig, jedoch für das Messgas undurchlässig ist. Der Schlauch 31 besteht bevorzugt aus einem sulfonierten Polytetrafluorethylen (PTFE). Der Schlauch 31 kann doppelwandig sein.

Das Messsystem 10 kann direkt über einem Industriebad oder aber auch in einem Labor direkt über einem Becherglas, also direkt über jeder beliebigen Flüssigkeitsoberfläche, zum Einsatz kommen. Es ist kein Pumpen der Prozessflüssigkeit notwendig.

Die optionale, über die Schnittstelle 25 lösbar angeschlossene Steuerung 26 ermöglicht die industrielle Umsetzung einer vollautomatischen Dosierung eines Biozids in Abhängigkeit von der gemessenen Keimkonzentration der Prozessflüssigkeit 11 bzw. in einem Prozessbad.

### Messmoden und Messzyklen:

Es sind zwei Messmoden möglich, nämlich eine Langzeitmessung (lange Messung) und eine Kurzzeitmessung (schnelle Messung):

### Langzeitmessung (lange Messung):

In KTL-Anlagen sind über 90% der gefundenen Mikroorganismen aerobe Mikroorganismen. Ein Hauptkeim bei KTL-Anlagen sind unabhängig von der Lacksorte die Bakterien der_Burkholderia Species. Diese Mikoorganismen verbrauchen bei ihrer Atmung Sauerstoff und produzieren dabei Kohlendioxid (Ausatmen von Kohlendioxid). Dieses Kohlendioxid wird im Überstand eines Behälters unter Verwendung des Gassensorarrays 12 und der Membranpumpe 14 gemessen und korreliert mit der Aktivität bzw. der Keimzahl der planktonischen Mikroorganismen. Entscheidend ist beim Aufbau die trichterförmige "Glocke" 15, die es ermöglicht, das Signal deutlich zu erhöhen und die Detektionsgrenze um ca. eine Zehnerpotenz zu verbessern.

### Kurzzeitmessung (schnelle Messung):

Aerobe Mikroorganismen verbrauchen bei ihrer Atmung Sauerstoff und produzieren dabei Kohlendioxid (Ausatmen von Kohlendioxid). Dieses Kohlendioxid wird teilweise in der fluiden Phase gelöst bzw. an den Festkörperpartikeln (in der Flüssigkeit/im Lack) adsorbiert. Bei gleichem Messaufbau und zusätzlichem Rührer 24 lässt sich dieses Kohlendioxid austreiben und messen. Dadurch ist Vorteil von kurzen Messzeiten unter 30 min realisierbar.

### Messzyklus Langzeitmessung:

Zunächst befindet sich der untere Rand 30 der Glocke 15 in einer Anhebestellung beispielsweise +15 cm über dem Flüssigkeitsspiegel bzw. der Flüssigkeitsoberfläche 17 des Bades/Behälters. In dieser Anhebestellung sind alle Sensoren (mindestens ein Kohlendioxid-Sensor, ein Feuchte-Sensor und ein Temperatursensor; beliebig erweiterbar, auch für andere Gase) und die Gas-Pumpe 14 mindestens schon 10 min im belüfteten Zustand in Betrieb.

Der Abstand der Glocke 15 von der Flüssigkeitsoberfläche 17 wird mittels eines Ultraschallsensors geregelt. Es erfolgt eine Abfrage der relativen Feuchte und der Kohlendioxid-Luftkonzentration. Die relative Feuchte muss vor der Absenkung der Glocke 15 unter 45% sein, sonst kondensiert Wasser auf der Sensorik. Die Kohlendioxid-Luftkonzentration muss unter einem Wert von 650 ppm liegen, damit diese auch noch bei kleinen Keimkonzentrationen, sprich Kohlendioxid-Änderungen, nicht im Rauschen untergehen.

Sind diese Vorgaben nicht erfüllt, muss eine Wartezeit bei ständigem Pumpen in Luft von 30min eingehalten werden. Sind danach die Werte (rel. Feuchte, Kohlendioxid-Luftkonzentration) immer noch nicht erfüllt, gibt die Anlage 10 ein Störsignal entweder auf die Steuerung 26 oder auf ein sonstiges Display und die Messung wird abgebrochen.

Erfüllen die Luftwerte die besagten Anforderungen, wird die Glocke 15 auf eine Absenkstellung 27 von beispielsweise -4 cm in die Prozessflüssigkeit 11 (Bad/Behälter), vorzugsweise geregelt, abgesenkt. Dabei wird im Überstand des Bades unter der Glocke 15 die Änderung der Kohlendioxid-Konzentration bestimmt. Nun wird über den Zeitraum von mehreren Stunden diese Kohlendioxid-Konzentration aufgezeichnet und von Algorithmen überwacht. Sollte sie innerhalb dieser Zeit einen bestimmten Wert überschreiten, wird die Messung abgebrochen, weil dann sehr hohe Keimwerte vorliegen.

Nach dem Ablaufen einer Messzeit von etwa 10 h wird die Glocke 15 wieder, vorzugsweise geregelt, in die Anhebestellung auf die Position mit +15 cm Abstand vom Badniveau (Flüssigkeitsoberfläche 17) hochgezogen.

Figur 2 zeigt die Kohlendioxid-Konzentrations-Verläufe von vier Langzeitmessungen, die im Abstand von jeweils etwa 24 h an einem Lack einer KTL-Anlage durchgeführt wurden. Jede Einzelmessung hat eine Dauer von 16,5 h. Bei der letzten Messung (obere Messkurve 32) wurde nach einem Zeitraum von 7,5 h Biozid zu dosiert. Deshalb fällt nach 7,5 h die Kohlendioxid-Konzentration ab. Die hierfür eingesetzte Messvorrichtung war ausgestattet mit drei NDIR-Sensoren (Nichtdispersive Infrarot-Sensoren) zur Messung der Kohlendioxid-Konzentration (Signalmittelung) in dem Lack.

Der jeweils erste Peak hat jeweils seine Ursache in dem in dem Lack gelösten Kohlendioxid (CO₂), das mittels des Rührers 24 freigesetzt wurde (siehe "Kurzzeitmessung (schnelle Messung)" und "Messzyklus schnelle Messung").

Für die Auswertung der Langzeitmessung bietet sich die Steigung zwischen der 6. und der 8. Stunde der Messzeit an.

### Algorithmus 1:

Linearer "Fit" (Gerade) durch diese beiden Punkte (6h und 8h) und Klassifizierung verschiedener Steigungsbereiche, also z.B. Steigung 0: Wenig Keime bis gar keine Keime bzw. Zuordnung zu einer Keimzahl.

Eine Schwierigkeit bei solchen Messungen an einer Anlage ist es, dass sehr viel Zeit aufgebracht werden muss, um den genauen Zeitpunkt einer Anlagenverkeimung zu erwischen. Sie tritt in diesem Fall alle 4-8 Wochen auf. D.h. man misst über die meiste Zeit Kurven wie in Figur 2 in der untersten Messkurve 33 gezeigt.

### Algorithmus 2:

Nehme den absoluten Wert der Konzentration nach einer bestimmten Anzahl von Stunden Messzeit (xh) und führe dann eine Klassifizierung durch.

### Algorithmus 3:

Dieser gilt nur für sehr hohe Konzentrationen, bei denen die gemessene Kohlendioxid-Konzentration aus dem Messbereich (0-2000ppm) der Gas-Sensoren herausläuft. Hier wäre die Zeit bis zum Erreichen der 2000ppm zu klassifizieren.

### Messzyklus schnelle Messung:

Bis zum Absenken der Glocke 15 ist der Messzyklus identisch. Nach dem Absenken der Glocke 15 in ihre Absenkstellung 27, wird der Rührer 24 beispielsweise mit 400U/min gestartet, um große Mengen an Kohlendioxid schnell austreiben zu können bzw. auszutreiben.

### Algorithmus 4:

Klassifizierung der Steigung und Absolutwert der Konzentration zum Zeitpunkt 10min.

### Algorithmus 5:

Klassifizierung des Signals durch den absoluten Wert der Konzentration am Scheitelpunkt der Kurve (f'(x)≅0).

Eine exakte Klassifizierung nach Keimzahlen kann je nach Anwendung erst nach einer Lernphase des Klassifikators erfolgen und ist natürlich abhängig vom untersuchten Fluid. Quantitative Aussagen lassen sich jedoch schnell treffen.

Zur Überwachung des Verkeimungsgrades der Prozessflüssigkeit 11 wird bevorzugt kontinuierlich, also unterbrechungsfrei, sowohl die Gas-Pumpe 14 betrieben als auch von dem wenigstens einen Gas-Sensor 12 ein Signal erzeugt. In vorgegebenen, vorzugsweise periodischen, zeitlichen Abständen werden die Langzeitmessungen über die vorgegebene lange Zeit durchgeführt. Bevorzugt zu Beginn jeder Langzeitmessung wird auch eine Kurzzeitmessung durchgeführt, wofür der Rührer 24 eine vorgegebene kurze Zeit betätigt wird. Kurzzeitmessungen können jedoch alternativ oder zusätzlich zu anderen Zeitpunkten während der Langzeitmessung oder auch zwischen aufeinander folgenden Langzeitmessungen durchgeführt werden. Beide Messungen können täglich im Wechsel durchgeführt werden.

Die Klassifizierung und Visualisierung erfolgt über einen Einplatinen-Computer, der bei einer Verbindung mit dem Internet die Daten in die Cloud hoch lädt, so dass die Daten bei einem vorhandenen Internetzugang überall abgerufen werden können. Eine Benachrichtigung auf ein Handy oder auf sonstige IOT-Anwendungen (IOT = Internet der Dinge) kann umgesetzt werden.

### BEZUGSZEICHENLISTE

- 10: Messgerät/Messsystem/Anlage
- 11: Prozessflüssigkeit
- 12: Sensorarray/Gassensorarray/Gas-Sensor
- 13: Auswertevorrichtung/Auswerteeinheit
- 14: Gas-Pumpe/Membranpumpe
- 15: Gasauffang-Hohlkörper/Gasauffangglocke/Glocke
- 16: Eintauch-Hohlraum
- 17: Flüssigkeitsoberfläche/Flüssigkeitsspiegel
- 18: Gasauffang-Hohlraum
- 19: Gas-Zuführleitung
- 20: Sensor-Vorrichtung
- 21: Gas-Rückführleitung
- 22: Hebe- und Senkvorrichtung
- 23: Absenkstellung
- 24: Rührer
- 25: Schnittstelle
- 26: Steuerung/Anlagensteuerung
- 27: Hals
- 28: Trichter
- 29: Trichterwandung
- 30: Rand
- 31: Schlauch
- 32: obere Messkurve
- 33: unterste Messkurve

## Patentansprüche

1. Messgerät (10) zur Bestimmung des Verkeimungsgrades von Prozessflüssigkeiten (11), das wenigstens einen Gas-Sensor (12) zur Messung der Gaskonzentration eines von etwaig oder tatsächlich in der Prozessflüssigkeit (11) enthaltenen aeroben Keimen produzierbaren oder produzierten Gases und eine damit verbundene Auswertevorrichtung (13) zur Auswertung eines von dem wenigstens einen Gas-Sensor (12) erzeugten Sensorsignals umfasst, das mit dem Verkeimungsgrad korreliert,
**dadurch gekennzeichnet,**
**dass** ein einen Eintauch-Hohlraum (16) aufweisender geometrischer Gasauffang-Hohlkörper (15) vorgesehen ist, der in die Prozessflüssigkeit (11) derart teilweise eintauchbar ist oder eingetaucht ist, dass in dem Gasauffang-Hohlkörper (15) oberhalb einer sich in dem Eintauch-Hohlraum (16) befindlichen Flüssigkeitsoberfläche (17) der Prozessflüssigkeit (11) ein Gasauffang-Hohlraum (18) zum Auffangen des aus der Prozessflüssigkeit (11) in die Umgebung austretenden Gases ausgebildet ist, und dass eine Gas-Zuführleitung (19) vorgesehen ist, deren eines Zuführleitungsende in den Gasauffang-Hohlraum (18) des Gasauffang-Hohlkörpers (15) mündet und deren anderes Zuführleitungsende in einen Vorrichtungs-Hohlraum einer den wenigstens einen Gas-Sensor (12) zum Messen der Konzentration des aus der Prozessflüssigkeit (11) ausgetretenen Gases enthaltenden Sensor-Vorrichtung (20) mündet, der in Gasverbindung mit dem wenigstens einem Gas-Sensor (12) steht, und dass eine aussschließlich an ihren beiden Rückführleitungsenden nach außen offene Gas-Rückführleitung (21) vorgesehen ist, deren eines Rückführleitungsende in den Vorrichtungs-Hohlraum der Sensor-Vorrichtung (20) mündet und deren anderes Rückführleitungsende in den Gasauffang-Hohlraum (18) des Gasauffang-Hohlkörpers (15) mündet, und dass eine Gas-Pumpe (14) zum Pumpen des Gases von dem Gasauffang-Hohlraum (18) des Gasauffang-Hohlkörpers (15) über den wenigstens einen Gas-Sensor (12) zurück in den Gasauffang-Hohlraum (18) des Gasauffang-Hohlkörpers (15) vorgesehen ist.

2. Messgerät nach Anspruch 1, **dadurch gekennzeichnet, dass** der Gasauffang-Hohlkörper (15) glockenförmig oder trichterförmig oder als Glocke oder als Trichter gestaltet ist.

3. Messgerät nach einem der vorstehenden Ansprüche, **dadurch gekennzeichnet, dass** ein Rührer (24) zur Homogenisierung der Prozessflüssigkeit und/oder ein Rührer (24) zum Austreiben des von den aeroben Keimen produzierten und in der Prozessflüssigkeit (11) gelösten Gases und/oder zum Austreiben des von den aeroben Keimen produzierten und an in der Prozessflüssigkeit (11) enthaltenen Festkörperpartikeln adsorbierten Gases vorgesehen ist.

4. Messgerät nach einem der vorstehenden Ansprüche, **dadurch gekennzeichnet, dass** eine steuerbare Hebe- und Senkvorrichtung (22) zum Anheben und Absenken des Gasauffang-Hohlkörpers (15), ggf. auch des Rührers (24), vorgesehen ist, mittels welcher der Gasauffang-Hohlkörper (15), ggf. auch der Rührer (24), von einer Anhebestellung außerhalb der Prozessflüssigkeit (11) in eine Absenkstellung (23) bewegbar ist, in welcher der Gasauffang-Hohlkörper (15) teilweise, ggf. auch der Rührer (24) zumindest teilweise, in die Prozessflüssigkeit (11) eingetaucht ist und in welcher die Gaskonzentration eines von den Keimen produzierten Gases mittels des wenigstens einen Gas-Sensors (12) messbar ist und mittels welcher der Gasauffang-Hohlkörper (15), ggf. auch der Rührer (24), von der Absenkstellung (23) in die Anhebestellung bewegbar ist.

5. Messgerät nach einem der vorstehenden Ansprüche, **dadurch gekennzeichnet, dass** die Hebe- und Senkvorrichtung (22) einen Abstands-Sensor zum Messen des Abstandes des Gasauffang-Hohlkörpers (15) zu der Flüssigkeitsoberfläche (17) der Prozessflüssigkeit (11) aufweist.

6. Messgerät nach Anspruch 4, **dadurch gekennzeichnet, dass** es sich bei dem Abstands-Sensor um einen Ultraschall-Sensor handelt.

7. Messgerät nach einem der vorstehenden Ansprüche, **dadurch gekennzeichnet, dass** die Auswertevorrichtung (13) mit einer Schnittstelle (25) zum Anschließen einer Steuerung (26) versehen ist, mittels welcher ein Biozid in Abhängigkeit von der mittels des wenigstens einen Gas-Sensors gemessenen Gaskonzentration des von den Keimen über der Zeit produzierten Gases in die Prozessflüssigkeit (11) dosierbar ist oder dass die Auswertevorrichtung (13) direkt oder über die Schnittstelle (25) mit einer Steuerung (26) verbunden ist, mittels welcher ein Biozid in Abhängigkeit von der mittels des wenigstens einen Gas-Sensors (12) gemessenen Gaskonzentration des von den Keimen über der Zeit produzierten Gases in die Prozessflüssigkeit (11) dosierbar ist.

8. Verfahren zur Bestimmung des Verkeimungsgrades von Prozessflüssigkeiten mittels des Messgeräts (10) nach einem der vorhergehenden Ansprüche, **dadurch gekennzeichnet, dass** die Gaskonzentration eines Gases, das von etwaig oder tatsächlich in einer Prozessflüssigkeit (11) enthaltenen Keimen produzierbar ist oder produziert wird, mittels des wenigstens einen Gas-Sensors (12) gemessen wird, der ein Sensorsignal erzeugt, das mit dem Verkeimungsgrad korreliert, wobei das Sensorsignal mittels der Auswertevorrichtung (13) ausgewertet wird,
wobei der Gasauffang-Hohlkörper (15) in die Prozessflüssigkeit (11) derart teilweise eingetaucht ist, dass in dem Gasauffang-Hohlkörper (15) oberhalb der sich in dem Eintauch-Hohlraum (16) befindlichen Flüssigkeitsoberfläche (17) der Prozessflüssigkeit (11) der Gasauffang-Hohlraum (18) zum Auffangen des aus der Prozessflüssigkeit (11) in die Umgebung austretenden Gases ausgebildet ist, wobei das aus der Prozessflüssigkeit (11) in die Umgebung ausgetretene Gas in dem Gasauffang-Hohlraum (18) aufgefangen und von dort mittels der Gas-Pumpe (14) über die Gas-Zuführleitung (19) dem wenigstens einen Gas-Sensor (12) zugeführt, über den wenigstens einen Gas-Sensor (12) geleitet und über die Gas-Rückführleitung (21) wieder in den Gasauffang-Hohlraum (18) des Gasauffang-Hohlkörpers (15) zurück gepumpt wird.

9. Verfahren nach Anspruch 8, **dadurch gekennzeichnet, dass** der Gasauffang-Hohlkörper (15) glockenförmig oder trichterförmig oder als Glocke oder als Trichter gestaltet ist.

10. Verfahren nach einem der Ansprüche 8 oder 9, **dadurch gekennzeichnet, dass** das von den aeroben Keimen produzierte und in der Prozessflüssigkeit (11) gelöste Gas und/oder dass das von den aeroben Keimen produzierte und an in der Prozessflüssigkeit (11) enthaltenen Festkörperpartikeln adsorbierte Gas mittels eines Rührers (24) ausgetrieben wird, wobei die Gaskonzentration des ausgetriebenen Gases mittels des wenigstens einen Gas-Sensors (12) gemessen wird.

11. Verfahren nach einem der Ansprüche 8 bis 10, **dadurch gekennzeichnet, dass** der Gasauffang-Hohlkörper (15), ggf. auch der Rührer (24), mittels einer steuerbaren Hebe- und Senkvorrichtung (22) von einer Absenkstellung (23), in welcher der Gasauffang-Hohlkörper (15) teilweise, ggf. auch der Rührer (24) zumindest teilweise, in die Prozessflüssigkeit (11) eingetaucht ist und in welcher die Gaskonzentration des von den Keimen produzierten Gases mittels des wenigstens einen Gas-Sensors (12) gemessen wird, in eine Anhebestellung überführbar ist, und umgekehrt, in welcher sich der Gasauffang-Hohlkörper (15), ggf. auch der Rührer (24), außerhalb der Prozessflüssigkeit (11) befindet.

12. Verfahren nach Anspruch 11, **dadurch gekennzeichnet, dass** der Gasauffang-Hohlkörper (15), ggf. auch der Rührer (24), bevor er sich in seiner Absenkstellung (23) befindet, mittels der steuerbaren Hebe- und Senkvorrichtung (22), ausgehend von der Anhebestellung, in welcher er sich außerhalb der Prozessflüssigkeit (11) befindet, in die Absenkstellung (22) bewegt wird, in welcher der Gasauffang-Hohlkörper (15) teilweise, ggf. auch der Rührer (24) zumindest teilweise, in die Prozessflüssigkeit (11) eingetaucht ist.

13. Verfahren nach einem der Ansprüche 8 bis 12, **dadurch gekennzeichnet, dass** die Auswertevorrichtung (13) direkt oder über eine Schnittstelle (25) mit einer Steuerung (26) verbunden ist, mittels welcher ein Biozid in Abhängigkeit von der mittels des wenigstens einen Gas-Sensors (12) gemessenen Gaskonzentration des von den Keimen über der Zeit produzierten Gases in die Prozessflüssigkeit (11) dosiert wird.

## Claims

1. Measuring apparatus (10) for determining the degree of bacterial contamination of process liquids (11), comprising at least one gas sensor (12) for measuring the gas concentration of a gas producible or produced by aerobic bacteria possibly or actually present in the process liquid (11) and an evaluating device (13), which is connected therewith, for evaluation of a sensor signal which is generated by the at least one gas sensor (12) and which is correlated with the degree of bacterial contamination, **characterised in that** a geometric gas collecting hollow body (15) is provided, which hollow body has an immersion cavity (16) and is partly immersible or immersed in the process liquid (11) in such a way that a gas collection cavity (18) for collecting the gas escaping from the process liquid (11) into the environment is formed in the gas collecting hollow body (15) above a liquid surface (17), which is present in the immersion cavity (16), of the process liquid (11), that a gas feed line (19) is provided, one feed line end of which communicates with the gas collection cavity (18) of the gas collecting hollow body (15) and the other feed line end of which communicates with a device cavity of a sensor device (20), which includes the at least one gas sensor (12) for measuring the concentration of the gas escaping from the process liquid (11) and which is in gas connection with the at least one gas sensor (12), that a gas return line (21), which is open to the outside exclusively at its two return line ends, is provided, one return line end of which communicates with the device cavity of the sensor device (20) and the other return line end of which communicates with the gas collection cavity (18) of the gas collecting hollow body (15), and that a gas pump (14) for pumping the gas from the gas collection cavity (18) of the gas collecting hollow body (15) by way of the at least one gas sensor (12) back to the gas collection cavity (18) of the gas collecting hollow body (15) is provided.

2. Measuring apparatus according to claim 1, **characterised in that** the gas collecting hollow body (15) is formed to be bell-shaped or funnel-shaped or as a bell or funnel.

3. Measuring apparatus according to one of the preceding claims, **characterised in that** an agitator (24) for homogenisation of the process liquid and/or an agitator (24) for expulsion of the gas produced by the aerobic bacteria and released into the process liquid and/or for expulsion of the gas produced by the aerobic bacteria and adsorbed at solid particles present in the process liquid (11) is or are provided.

4. Measuring apparatus according to any one of the preceding claims, **characterised in that** a controllable raising and lowering device (22) for raising and lowering the gas collecting hollow body (15) and optionally the agitator (24) is provided, by means of which the gas collecting hollow body (15) and optionally the agitator (24) are movable from a raised setting outside the process liquid (11) to a lowered setting (23), in which the gas collecting hollow body (15) is partly and optionally the agitator (24) at least partly immersed in the process liquid (11) and in which the gas concentration of a gas produced by the bacteria is measurable by way of the at least one gas sensor (12), and by means of which the gas collecting hollow body (15) and optionally the agitator (24) are movable from the lowered setting (23) to the raised setting.

5. Measuring apparatus according to any one of the preceding claims, **characterised in that** the raising and lowering device (22) comprises a distance sensor for measuring the spacing of the gas collecting hollow body (15) from the liquid surface (17) of the process liquid (11).

6. Measuring apparatus according to claim 4, **characterised in that** the distance sensor is an ultrasonic sensor.

7. Measuring apparatus according to any one of the preceding claims, **characterised in that** the evaluating device (13) is provided with an interface (25) for connection with a control (26) by means of which a biocide can be admetered to the process liquid (11) in dependence on the gas concentration, which is measured by way of the at least one gas sensor, of the gas produced over time by the bacteria or that the evaluating device (13) is connected directly or via the interface (25) with a control (26) by means of which a biocide can be admetered to the process liquid (11) in dependence on the gas concentration, which is measured by way of the at least one gas sensor (12), of the gas produced over time by the bacteria.

8. Method of determining the degree of bacterial contamination of process liquids by means of the measuring apparatus (10) according to any one of the preceding claims, **characterised in that** the gas concentration of a gas producible or produced by bacteria possibly or actually present in a process liquid (11) is measured by way of the at least one gas sensor (12) which produces a sensor signal correlated with the degree of bacterial contamination, wherein the sensor signal is evaluated by means of the evaluating device (13), wherein the gas collecting hollow body (15) is partly immersed in the process liquid (11) in such a way that the gas collection cavity (18) for collecting the gas escaping from the process liquid (11) into the environment is formed in the gas collecting hollow body (15) above the liquid surface (17), which is present in the immersion cavity (16), of the process liquid (11), wherein the gas escaping from the process liquid (11) into the environment is collected in the gas collection cavity (18) and from there fed by means of the gas pump (14) via the gas feed line (19) to the at least one gas sensor (12), conducted via the at least one gas sensor (12) and pumped by way of the gas return line (21) back again to the gas collection cavity (18) of the gas collecting hollow body (15).

9. Method according to claim 8, **characterised in that** the gas collecting hollow body (15) is funnel-shaped or bell-shaped or is formed as a bell or funnel.

10. Method according to one of claims 8 and 9, **characterised in that** the gas produced by the aerobic bacteria and released into the process liquid (11) and/or the gas produced by the aerobic bacteria and adsorbed at solid particles present in the process liquid (11) is or are expelled by means of an agitator (24), wherein the gas concentration of the expelled gas is measured by way of the at least one gas sensor (12).

11. Method according to any one of claims 8 to 10, **characterised in that** the gas collecting hollow body (15) and optionally the agitator (24) are transferrable by means of a controllable raising and lower device (22) from a lowered setting (23), in which the gas collecting hollow body (15) is partly and optionally the agitator (24) at least partly immersed in the process liquid (11) and in which the gas concentration of the gas produced by the bacteria is measured by way of the at least one gas sensor (12), to a raised setting, in which the gas collecting hollow body (15) and optionally the agitator (24) are disposed outside the process liquid (11), and conversely.

12. Method according to claim 11, **characterised in that** the gas collecting hollow body (15) and optionally the agitator (24) before being disposed in the lowered setting (23) thereof are moved by means of the controllable raising and lowering device (22), starting from the raised setting in which they are disposed outside the process liquid (11), to the lowered setting (22) in which the gas collecting hollow body (15) is partly and optionally the agitator (24) at least partly immersed in the process liquid (11).

13. Method according to any one of claims 8 to 12, **characterised in that** the evaluating device (13) is connected directly or by way of an interface (25) with a control (26), by means of which a biocide is admetered to the process liquid (11) in dependence on the gas concentration, which is measured by way of the at least one gas sensor (12), of the gas produced over time by the bacteria.

## Revendications

1. Appareil de mesure (10) pour déterminer le degré de contamination par des germes de liquides de processus (11), qui comprend au moins un capteur de gaz (12) pour mesurer la concentration en gaz d'un gaz qui peut être produit ou est produit par des germes aérobies éventuellement ou effectivement contenus dans le liquide de processus (11), et un dispositif d'évaluation (13) relié à celui-ci pour évaluer un signal de capteur qui est généré par ledit au moins un capteur de gaz (12) et qui est corrélé au degré de contamination par des germes,
caractérisé en ce en ce
qu'il est prévu un corps creux géométrique de collecte de gaz (15) présentant une cavité d'immersion (16), qui peut être ou est en partie immergé dans le liquide de processus (11) de telle sorte qu'une cavité de collecte de gaz (18) destinée à collecter le gaz s'échappant du liquide de processus (11) dans l'environnement soit formée dans le corps creux de collecte de gaz (15), au-dessus d'une surface de liquide (17) du liquide de processus (11) se trouvant dans la cavité d'immersion (16), et qu'une conduite d'amenée de gaz (19) est prévue, dont une extrémité de conduite d'amenée débouche dans la cavité de collecte de gaz (18) du corps creux de collecte de gaz (15) et dont l'autre extrémité de conduite d'amenée débouche dans une cavité de dispositif d'un dispositif capteur (20) contenant ledit au moins un capteur de gaz (12) pour mesurer la concentration du gaz s'échappant du liquide de processus (11), lequel dispositif capteur est en communication gazeuse avec ledit au moins un capteur de gaz (12), et qu'il est prévu une conduite de retour de gaz (21) ouverte vers l'extérieur exclusivement à ses deux extrémités de conduite de retour, dont une extrémité de conduite de retour débouche dans la cavité de dispositif du dispositif capteur (20) et l'autre extrémité de conduite de retour débouche dans la cavité de collecte de gaz (18) du corps creux de collecte de gaz (15), et qu'une pompe à gaz (14) est prévue pour pomper le gaz de la cavité de collecte de gaz (18) du corps creux de collecte de gaz (15) et le ramener dans la cavité de collecte de gaz (18) du corps creux de collecte de gaz (15) en passant par ledit au moins un capteur de gaz (12).

2. Appareil de mesure selon la revendication 1, **caractérisé en ce que** le corps creux de collecte de gaz (15) est conçu en forme de cloche ou d'entonnoir ou comme une cloche ou un entonnoir.

3. Appareil de mesure selon l'une des revendications précédentes, **caractérisé en ce qu'**il est prévu un agitateur (24) pour homogénéiser le liquide de processus et/ou un agitateur (24) pour expulser le gaz produit par les germes aérobies et dissous dans le liquide de processus (11) et/ou pour expulser le gaz produit par les germes aérobies et adsorbé sur des particules solides contenues dans le liquide de processus (11).

4. Appareil de mesure selon l'une des revendications précédentes, **caractérisé en ce qu'**il est prévu un dispositif de levage et d'abaissement commandable (22) pour lever et abaisser le corps creux de collecte de gaz (15), le cas échéant aussi l'agitateur (24), au moyen duquel le corps creux de collecte de gaz (15), le cas échéant aussi l'agitateur (24), peut être déplacé d'une position levée en dehors du liquide de processus (11) dans une position abaissée (23) dans laquelle le corps creux de collecte de gaz (15) est en partie, le cas échéant aussi l'agitateur (24) au moins en partie, immergé dans le liquide de processus (11) et dans laquelle la concentration en gaz d'un gaz produit par les germes peut être mesurée au moyen dudit au moins un capteur de gaz (12) et au moyen duquel le corps creux de collecte de gaz (15), le cas échéant aussi l'agitateur (24), peut être déplacé de la position abaissée (23) dans la position levée.

5. Appareil de mesure selon l'une des revendications précédentes, **caractérisé en ce que** le dispositif de levage et d'abaissement (22) présente un capteur de distance pour mesurer la distance entre le corps creux de collecte de gaz (15) et la surface de liquide (17) du liquide de processus (11).

6. Appareil de mesure selon la revendication 4, **caractérisé en ce que** le capteur de distance est un capteur à ultrasons.

7. Appareil de mesure selon l'une des revendications précédentes, **caractérisé en ce que** le dispositif d'évaluation (13) est pourvu d'une interface (25) pour la connexion d'une unité de commande (26), au moyen de laquelle un biocide peut être dosé dans le liquide de processus (11) en fonction de la concentration en gaz, mesurée au moyen dudit au moins un capteur de gaz, du gaz produit par les germes au cours du temps, ou que le dispositif d'évaluation (13) est relié, directement ou par l'intermédiaire de l'interface (25), à une unité de commande (26) au moyen de laquelle un biocide peut être dosé dans le liquide de processus (11) en fonction de la concentration en gaz, mesurée au moyen dudit au moins un capteur de gaz (12), du gaz produit par les germes au cours du temps.

8. Procédé pour déterminer le degré de contamination par des germes de liquides de processus au moyen de l'appareil de mesure (10) selon l'une des revendications précédentes, **caractérisé en ce que** la concentration en gaz d'un gaz qui peut être produit ou est produit par des germes éventuellement ou effectivement contenus dans un liquide de processus (11), est mesurée au moyen dudit au moins un capteur de gaz (12) qui génère un signal de capteur qui est corrélé au degré de contamination par des germes, le signal de capteur étant évalué au moyen du dispositif d'évaluation (13), le corps creux de collecte de gaz (15) étant partiellement immergé dans le liquide de processus (11) de telle sorte que la cavité de collecte de gaz (18) destinée à collecter le gaz s'échappant du liquide de processus (11) dans l'environnement soit formée dans le corps creux de collecte de gaz (15), au-dessus de la surface de liquide (17) du liquide de processus (11) se trouvant dans la cavité d'immersion (16), le gaz s'échappant du liquide de processus (11) dans l'environnement étant collecté dans la cavité de collecte de gaz (18) et de là amené au moyen de la pompe à gaz (14), par la conduite d'amenée de gaz (19), audit au moins un capteur de gaz (12), conduit par ledit au moins un capteur de gaz (12) et ramené dans la cavité de collecte de gaz (18) du corps creux de collecte de gaz (15) par la conduite de retour de gaz (21).

9. Procédé selon la revendication 8, **caractérisé en ce que** le corps creux de collecte de gaz (15) est conçu en forme de cloche ou d'entonnoir ou comme une cloche ou un entonnoir.

10. Procédé selon l'une des revendications 8 ou 9, **caractérisé en ce que** le gaz produit par les germes aérobies et dissous dans le liquide de processus (11) et/ou que le gaz produit par les germes aérobies et adsorbé sur des particules solides contenues dans le liquide de processus (11) est expulsé au moyen d'un agitateur (24), la concentration en gaz du gaz expulsé étant mesurée au moyen dudit au moins un capteur de gaz (12).

11. Procédé selon l'une des revendications 8 à 10, **caractérisé en ce que** le corps creux de collecte de gaz (15), le cas échéant aussi l'agitateur (24), peut être transféré, au moyen d'un dispositif de levage et d'abaissement commandable (22), d'une position abaissée (23) dans laquelle le corps creux de collecte de gaz (15) est en partie, le cas échéant aussi l'agitateur (24) au moins en partie, immergé dans le liquide de processus (11) et dans laquelle la concentration en gaz du gaz produit par les germes est mesurée au moyen dudit au moins un capteur de gaz (12), dans une position levée, dans laquelle le corps creux de collecte de gaz (15), le cas échéant aussi l'agitateur (24), se trouve en dehors du liquide de processus (11), et inversement.

12. Procédé selon la revendication 11, **caractérisé en ce que** le corps creux de collecte de gaz (15), le cas échéant aussi l'agitateur (24), avant qu'il ne se trouve dans sa position abaissée (23), est déplacé au moyen du dispositif de levage et d'abaissement commandable (22), de la position levée dans laquelle il se trouve en dehors du liquide de processus (11), dans la position abaissée (22) dans laquelle le corps creux de collecte de gaz (15) est en partie, le cas échéant aussi l'agitateur (24) au moins en partie, immergé dans le liquide de processus (11).

13. Procédé selon l'une des revendications 8 à 12, **caractérisé en ce que** le dispositif d'évaluation (13) est relié directement ou par l'intermédiaire d'une interface (25) à une unité de commande (26) au moyen de laquelle un biocide est dosé dans le liquide de processus (11) en fonction de la concentration en gaz, mesurée au moyen dudit au moins un capteur de gaz (12), du gaz produit par les germes au cours du temps.
